# EUROPEAN PATENT SPECIFICATION

(11) **EP 2 435 123 B1**
(45) Date of publication and mention of the grant of the patent: **16.05.2018**
(21) Application number: 10781082.2
(22) Date of filing: 25.05.2010
(51) Int. Cl.: A61B 34/35, A61M 25/01, A61B 34/30

(54) **REMOTE MANIPULATOR DEVICE**
FERNMANIPULATORVORRICHTUNG
DISPOSITIF DE MANIPULATION À DISTANCE

(30) Priority: 25.05.2009 US 180926 P
(43) Date of publication of application: 04.04.2012
(73) Proprietor: Stereotaxis, Inc., St. Louis, MO 63108 (US)
(72) Inventor: KIDD, Brian L., St. Louis, MO 63108 (US); KASTELEIN, Nathan, St. Louis, MO 63108 (US)
(74) Representative: Hedges, Martin Nicholas
(86) International application number: PCT/US2010/036052
(87) International publication number: WO 2010/138499

(56) References cited:
- EP-A1- 0 116 526
- WO-A1-96/39944
- WO-A2-2005/117688
- WO-A2-2007/008967
- WO-A2-2008/101228
- US-A1- 2003 144 649
- US-A1- 2006 058 738
- US-A1- 2008 009 791
- US-A1- 2009 054 835
- US-A1- 2009 054 835
- US-A1- 2009 105 639
- US-A1- 2009 105 639
- US-B1- 6 474 377

## Description

This invention as defined in claim 1, with preferred embodiments as defined in the dependent claims, relates to automating the operation of medical devices.

Significant progress has been made in automating the navigation of medical devices in the body. Remote navigation systems, such as the Niobe® magnetic navigation system available from Stereotaxis, Inc., St. Louis, MO, allows a physician to remotely orient the distal end of a medical device in the body. More recently, an automated advancer for advancing and retracting the device in the body has also become available, allowing more fully automated catheter navigation systems. However, a practical means of completely automating (under the supervision of a physician) the operation of medical devices, whereby a medical device can be automatically navigated to a particular location and then operated to perform some diagnostic or therapeutic procedure has not been available. This is particularly true with respect to automating the operation of conventional manually operated medical devices.
US 2009/054835, which is considered to represent the closest prior art, discloses a system for remotely manipulating an elongate medical device of the type having a distal end adapted to be introduced into a patient and a handle at the proximal end with rotatable and translatable controls for manipulating the device, the system comprising: a device driver positionable adjacent the patient; a device interface releasably mounted on the device driver; and a control for selectively operating the device driver to advance and retract the device driver relative to the patient to advance and retract the distal end of the medical device in the patient

Other prior art systems are disclosed in US2009/105639, WO2008/101228 and US 2006/058738.

### SUMMARY

Embodiments of the present invention provide a remote manipulator that not only can manipulate a conventional catheter, but can operate its controls. This not only allows the catheter to be remotely navigated, but also to be remotely operated. This allows a physician to conduct the procedure away from the patient, and also permits the complete automation of the procedure, with a computer navigating the catheter and operating the catheter without the need for human intervention.

According to the present there is provided a system for remotely manipulating an elongated medical device according to claim 1.

Further areas of applicability will become apparent from the description provided herein. The description and specific examples in this summary are intended for purposes of illustration only and are not intended to limit the scope of the present disclosure.

### DRAWINGS

In order that the invention may be well understood, there will now be described an embodiment thereof, given by way of example, reference being made to the accompanying drawings, in which:
Fig. 1 is a front perspective view of a preferred embodiment of a remote manipulator device in accordance with the principles of this invention;
Fig. 2 is a rear perspective view of the preferred embodiment of the remote manipulator device, with the drive cables removed for clarity;
Fig. 3 is a top plan view of the preferred embodiment of the remote manipulator device, with the drive cables removed for clarity;
Fig. 4 is a rear elevation view of the preferred embodiment of the remote manipulator device, with the drive cables removed for clarity;
Fig. 5 is a left side elevation view of the preferred embodiment of the remote manipulator device, with the drive cables removed for clarity;
Fig. 6 is an exploded view of the preferred embodiment of the remote manipulator device;
Fig. 7 is a front perspective view of the preferred embodiment of the remote manipulator device, with the device driver removed for clarity;
Fig. 8 is a rear perspective view of the preferred embodiment of the remote manipulator device, with the device driver removed for clarity;
Fig. 9 is a left side elevation view of the preferred embodiment of the remote manipulator device, with the device driver removed for clarity;
Fig. 10 is a top plan view of the preferred embodiment of the remote manipulator device, with the device driver removed for clarity;
Fig. 11 is a front elevation view of the preferred embodiment of the remote manipulator device, with the device driver removed for clarity;
Fig. 12 is a rear perspective view of the preferred embodiment, with the cover removed from the controller;
Fig. 13 is a perspective view of the device driver of the preferred embodiment;
Fig. 14 is a side elevation view of the device driver of the preferred embodiment;
Fig. 15 is an front end elevation view of the device driver of the preferred embodiment;
Fig. 16 is a rear end elevation view of the device driver of the preferred embodiment;
Fig. 17 is a top plan view of the device driver of the preferred embodiment;
Fig. 18 is a perspective view of the body of the device driver, with the upper and lower members separated to show the details of construction;
Fig. 19 is a perspective view of the body of the device driver, with the upper member removed to show the details of construction;
Fig. 20 is an exploded perspective view of the body of the device driver, with the upper member removed;
Fig. 21 is an exploded perspective view of the body of the device driver, with parts removed to show details of construction;
Fig. 22 is a perspective view of the device interface of the preferred embodiment;
Fig. 23 is a top plan view of the device interface of the preferred embodiment;
Fig. 24 is a side elevation view of the device interface of the preferred embodiment;
Fig. 25 is a bottom plan view of the device interface of the preferred embodiment;
Fig. 26 is an exploded perspective view of the device interface of the preferred embodiment;
Fig. 27 is a perspective view of one of the clamps used in the device interface of the preferred embodiment;
Fig. 28 is a rear elevation view of the clamp;
Fig. 29 is a front elevation view of the clamp;
Fig. 30 is a top plan view of the clamp;
Fig. 31 is a side elevation view of the clamp, showing the hinged connection between the base and cover;
Fig 32 is an exploded perspective view of the clamp;
Fig. 33 is a perspective view of the split ring adapter used in the clamp;
Fig. 34 is a front elevation view of the split ring adapter;
Fig, 35 is a side elevation view of the split ring adapter;
Fig. 36 is an exploded perspective view of the split ring adapter;
Fig. 37 is a perspective view showing the support installed on the bed;
Fig. 38 is a perspective view showing the mounting of the bracket and controller on the base plate of the support;
Fig. 39 is a perspective view of the proximal end of a telescoping catheter support;
Fig. 40A is a perspective view of a medical device being placed in the clamps;
Fig. 40B is a perspective view of the covers of the clamps being closed over a medical device being placed in the clamps; and
Fig. 40C is a perspective view of the covers of the clamps being latched to secure a medical device being placed in the clamps.

Corresponding reference numerals indicate corresponding parts throughout the several views of the drawings.

### DETAILED DESCRIPTION

Example embodiments will now be described more fully with reference to the accompanying drawings.

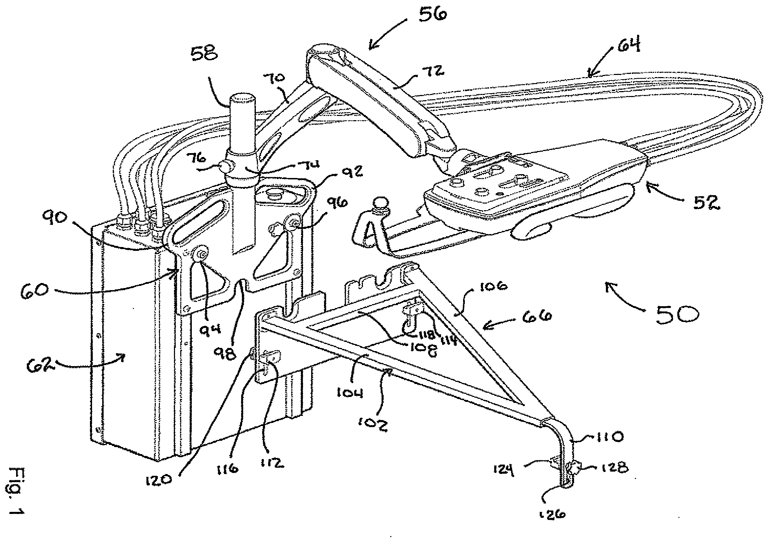

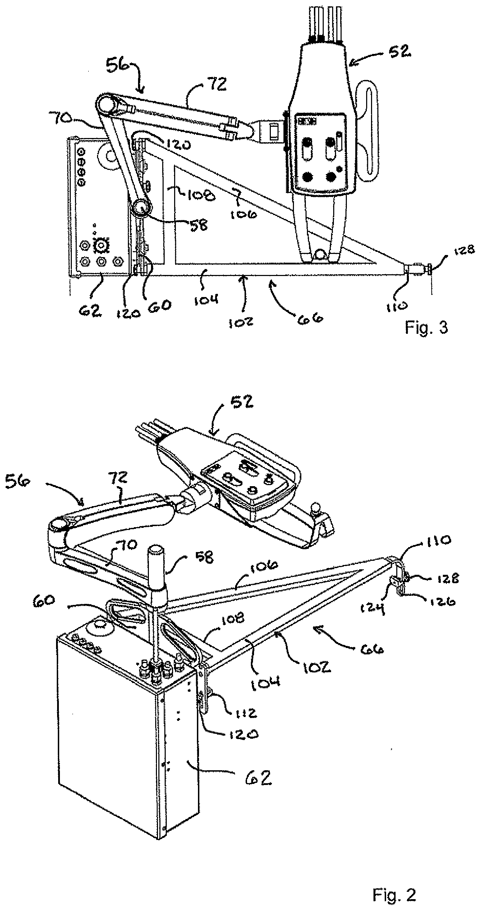

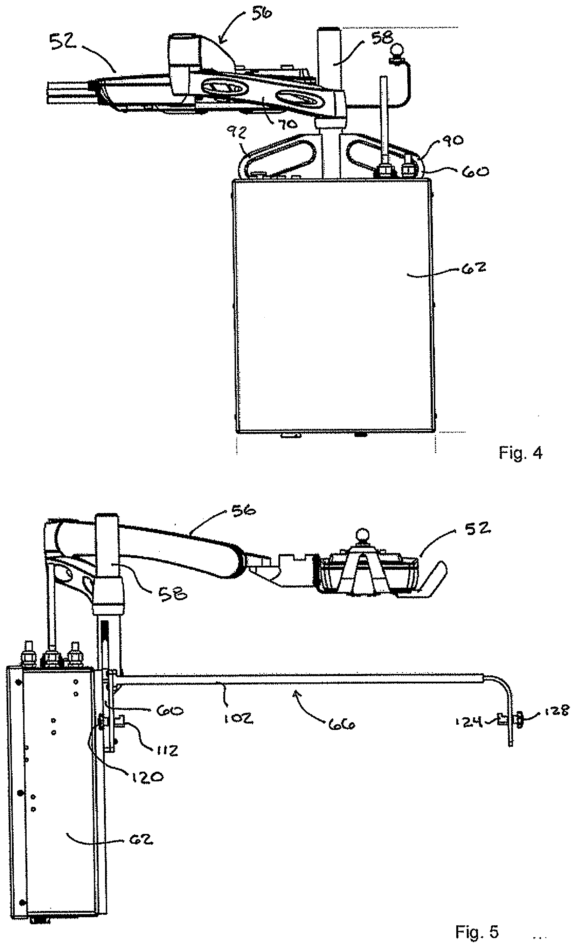

A preferred embodiment of a remote manipulator device constructed according to the principles of this invention is indicated generally as 50 in Figs. 1-5. The remote manipulator 50 is adapted to engage and operate a medical device. The medical device may be, for example, an electrophysiology catheter of the type comprising an elongate sheath having a handle at its proximal end, and an electrophysiology wire extending through the sheath and out the distal end, forming a loop. The handle preferably has controls for manipulating the distal end of the wire, for example a translatable control for bending the distal end, and a rotatable control for expanding and contracting the loop. While this preferred embodiment is described with respect to an electrophysiology catheter, this invention is not limited to electrophysiology catheters and applies to any medical device with an elongate portion, and a handle at the proximal end that can be manipulated to position and operate the distal end of the elongate portion of the medical device.

As shown in the Figs. 1-5, the remote manipulator 50 comprises a device driver 52 for mounting and operating a device interface 54 (not shown in Figs. 1 - 5), which in this preferred embodiment is both replaceable and disposable. The device driver 52 is mounted on the distal end of an articulated arm 56, whose proximal end is mounted on a post 58 on bracket 60. A controller 62, which can be mounted to the bracket 60, is connected to the device driver 52 by a plurality of flexible drive cables 64 to operate the device driver 52. A platform 66, attachable to the patient bed (not shown in Figs. 1-5), can mount the bracket 60 and/or the controller 62.

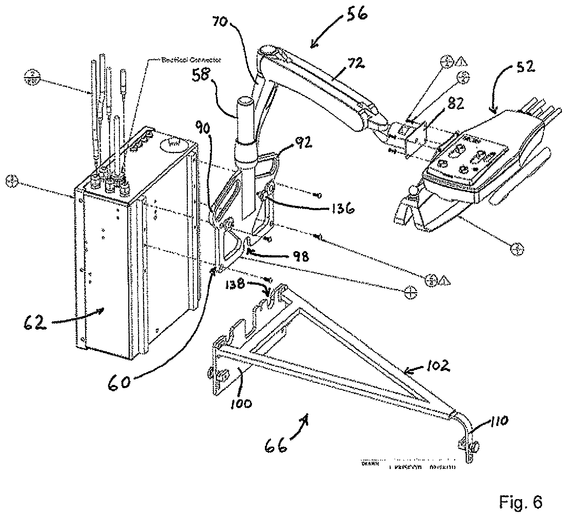

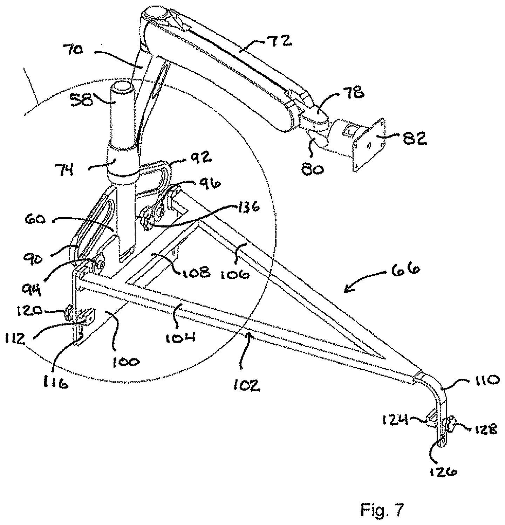

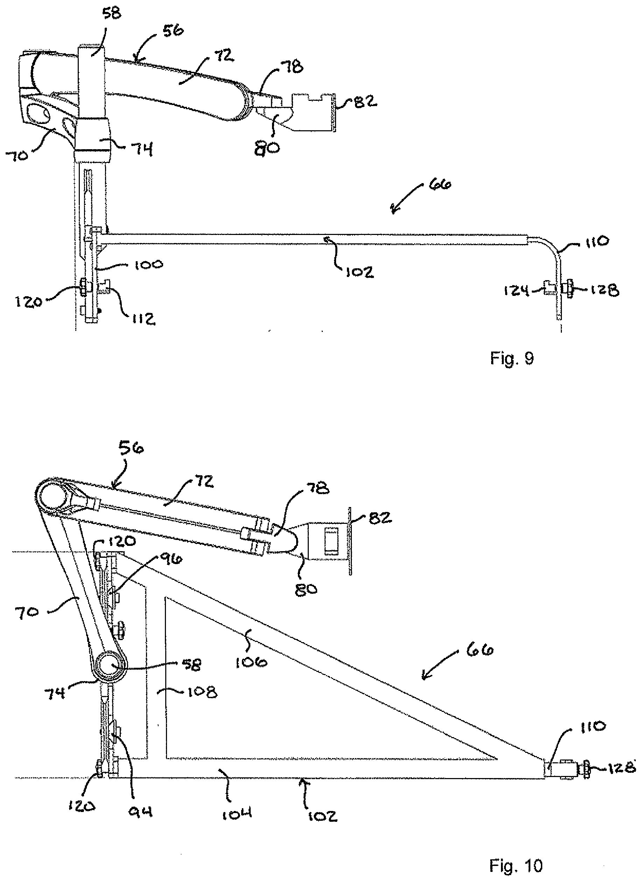

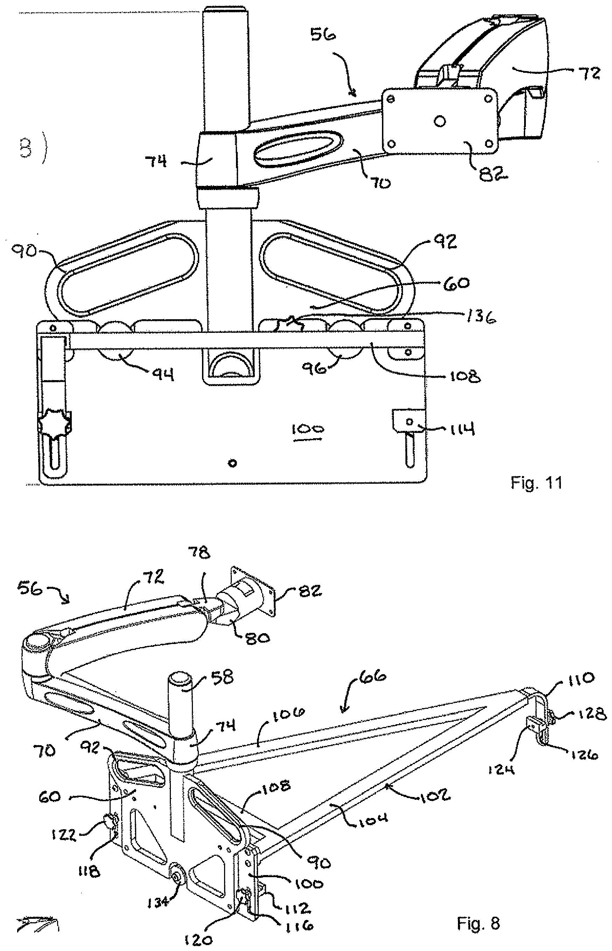

As shown in Fig. 6-11, the articulated arm 56 comprises proximal and distal sections 70 and 72. Proximal section 70 has a collar 74 at its proximal end for mounting the proximal section 70 on the post 58. The proximal section 70 can translate up and down, and rotate around, the post 58. A locking screw 76 allows the collar and thus, the proximal section 70 to be releasably locked relative to the post 58. The distal end of the proximal section 70 is pivotally connected to the proximal end of the distal section 72. A lock (not shown) can be provided to releasably lock the proximal and distal sections 70 and 72 relative to each other. A first wrist element 78 is pivotally mounted to the distal end of the distal section 72 to pivot about a first axis generally perpendicular to the longitudinal axis of the distal section. A second wrist element 80 is pivotally mounted to the first wrist element to pivot about a second axis generally perpendicular to the first axis. A mounting plate 82 is rotatably mounted to the second wrist element 80 to rotate about a third axis, perpendicular to the first axis. The wrists pivoting about the first and second axis and the mounting plate rotating about the third axis are selectively releasably lockable, to releasably secure the articulated arm in a desired configuration with the device driver 52 in a desired location.

As also shown in Figs. 6-11, the bracket 60 has hand loops 90 and 92 thereon. Pins 94 and 96 with enlarged conical heads project from the front face of the bracket 60. There is a semi-circular notch 98 in the bottom of the bracket 60.

As also shown in Figs. 6-11, the platform 66 comprises a base plate 100, and a generally right-triangular frame 102, comprising legs 104 and 106, extending from the base plate 100 and leg 108 extending between the legs 104 and 106. A generally L-shaped bracket 110 telescopes from the leg 104 of the frame 102. Cleats 112 and 114 are mounted in slots 116 and 118 in the base plate 100 with threaded bolts 120 and 122 which have knurled heads that allow the cleats to be tightened against the side rail typically found on a patient bed. Similarly, a cleat 124 is mounted in slot 126 in the end of L-shaped bracket 110 with a threaded bolt 128 with a knurled head that allows the cleat to be tightened against the side rail of the patient bed. The top edge of the base plate 100 has semi-circular notches 130 and 132 for engaging the pins 94 and 96 on the bracket, and the back face of the base plate has a projecting pin 134 for engaging the semi-circular notch 98 in the bracket 60. A bolt 136 on the bracket 60 can be tightened to engage a slot 138 on the base plate 100 to secure the bracket and base plate 100.

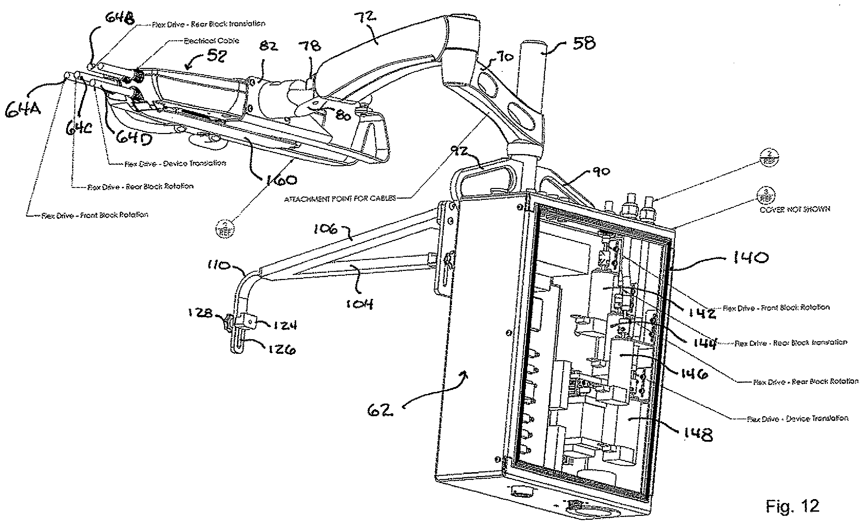

The controller 62 can be attached to the bracket 60 and mounted on the platform 66. As shown in Fig. 12, the controller includes a housing 140 with four drives: a drive 142 for operating a first clamp driver on the device driver 52; a drive 144 for operating the second clamp translation driver on the device driver; a drive 146 for operating a second clamp driver on the device driver; and a drive 148 for operating the translation driver on the device driver for translating the device, as will be described in more detail below. Flexible drive cables 64 connect each drive in the controller with its respective driver in the device drive 52.

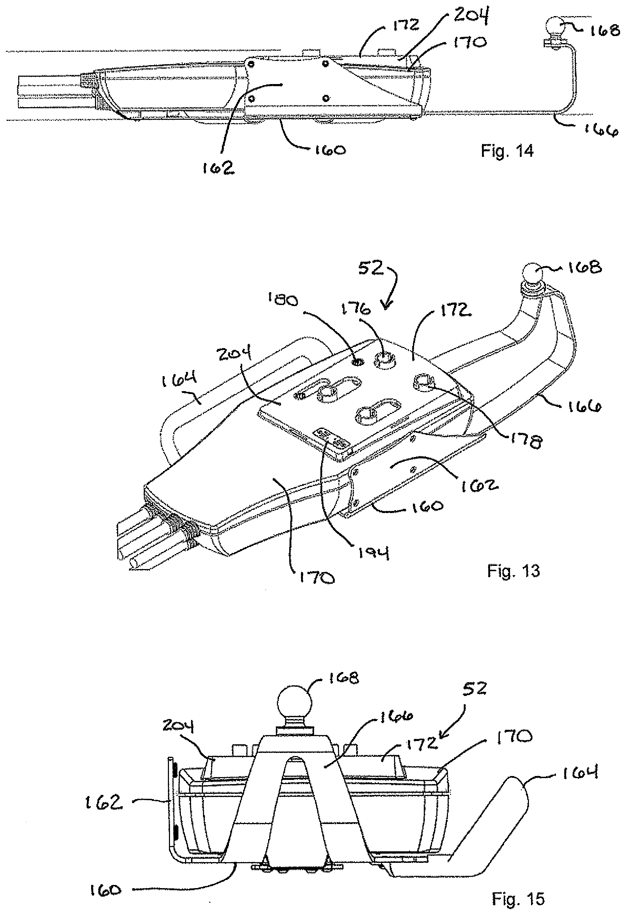

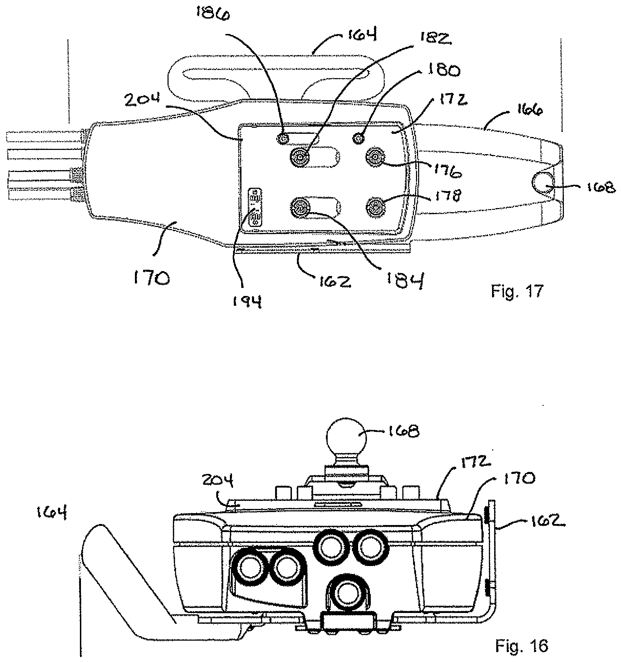

As shown in Figs. 13-21, the device driver 52 comprises a base 160 having a mount 162 for attaching the device driver to the mounting plate 82 on the end of the articulated arm 56. A hand loop 164 on the opposite side of the base 160 from the mount 162 can be provided for gripping the device driver 52 to reposition it. A generally V-shaped bracket 166 projects from the front of the base 160, the end of the bracket is bent upwardly and has a fixture 168 mounted thereon for mounting a sheath, as described in more detail below. The device driver 52 further comprises a body 170 mounted on the base 160 to translate forwardly and rearwardly with respect to the base.

The top surface of the body 170 has a platform 172 for mounting a device interface 54 thereon. As described below, the device interface 54 has one or more clamps 174 (not shown in Figs. 13-21) for engaging and operating the handle of an elongate medical device. The platform 172 has a first pair of mounting sockets 176 and 178 for engaging a clamp 174 on the device interface 54, and a drive socket 180 for receiving and engaging a drive shaft of the clamp to operate the clamp. In this preferred embodiment, the platform 172 also has a second pair of mounting sockets 182 and 184 for engaging a second clamp 174 on the device interface 54, and a drive socket 186 for receiving and engaging a drive shaft of the second clamp to operate the second clamp. The second pair of sockets 182 and 184, and the drive socket 186 are disposed in slots so that they can translate relative to the first set of sockets 176 and 178, to accommodate relative movement between the clamps 174 on the device interface 54, as will be described in more detail below. There is an electrical contact pad 194 on the platform 172 for making electrical contact with a corresponding electrical contact pad 196 on the device interface 54.

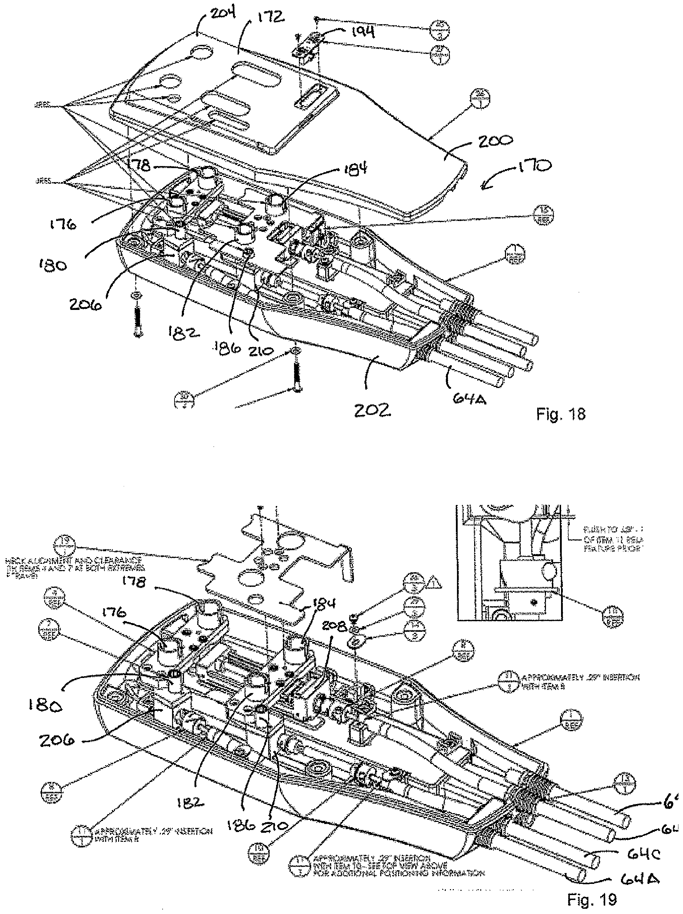

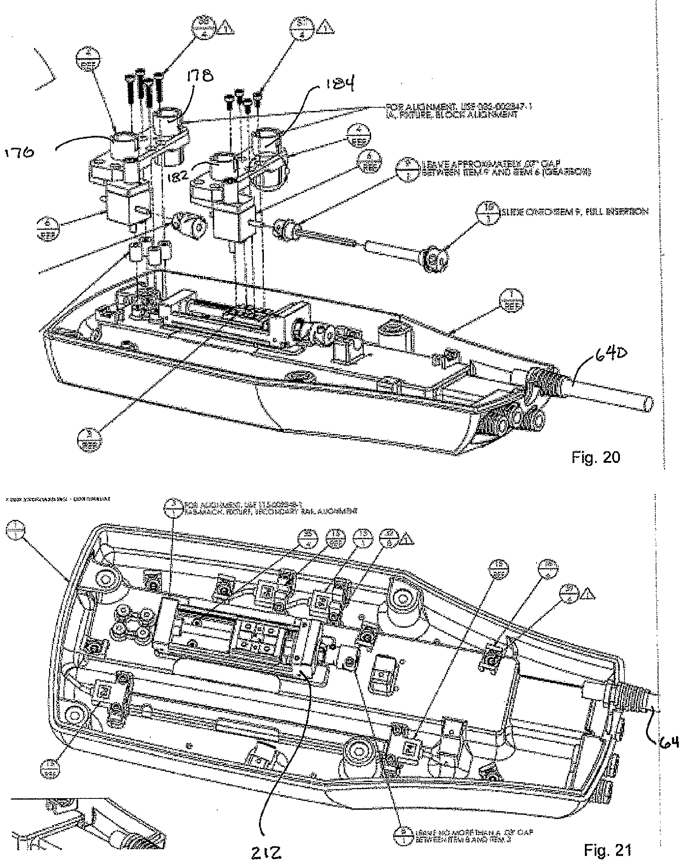

As shown in detail in Fig. 18, the body 170 comprises upper and lower housing members 200 and 202. The platform 172 is formed in the top of the upper housing member 200, and comprises a raised platform area 204 with openings for the sockets 176 and 178 and 182 and 184, as well as contact pad 194. As shown in Fig. 19, in the lower housing member 202, the distal end of flexible drive cable 64A, connects the drive 142 to a transmission 206 for turning socket 180 to operate a first clamp 174 on a device interface 54 seated on the platform 172. The distal end of a cable 64B connects the drive 144 to a screw-driven translation mechanism 208 for translating the second pair of sockets 182 and 184 relative to first pair or sockets 176 and 178. The distal end of cable 64C connects the drive 146 to a transmission 210 for turning socket 186 to operate a second clamp 174 on a device interface 54. Lastly, a flexible drive cable 64D connects the drive 148 to a screw translation mechanism 212 (Fig. 21) for moving the body 170 relative to the base 160.

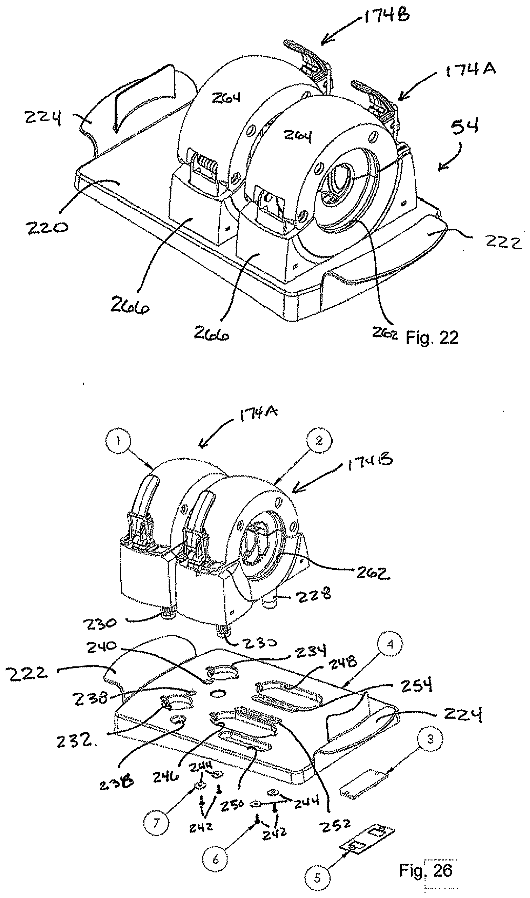

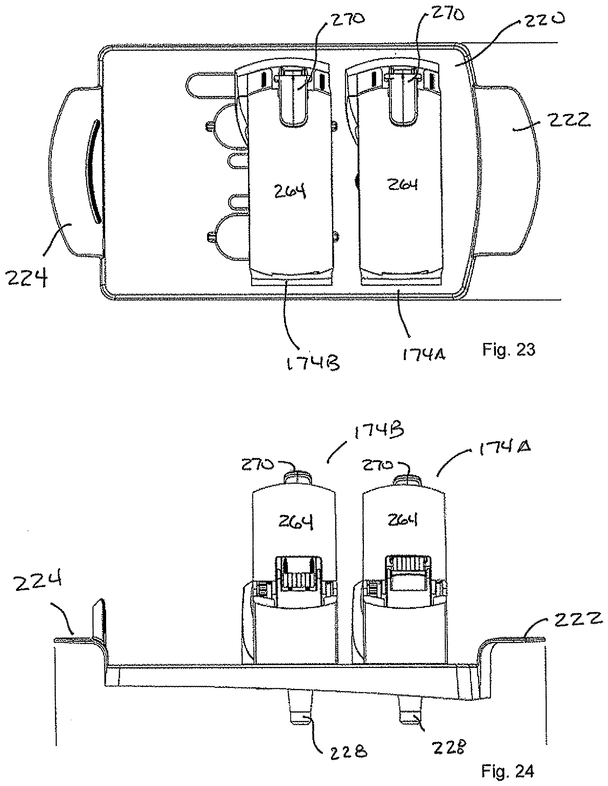

As shown in Figs. 22 - 26, the device interface 54 comprises a tray 220 that is adapted to fit onto the raised platform 204 forming the platform 172 on the device driver 52. The tray 220 has a generally rectangular shape, but preferably has a curved side or other feature that prevents the tray from being installed on the device driver 52 incorrectly. There are handles 222 and 224 at either end of the tray 220 to facilitate handling the tray. One or more clamps 174 are mounted on the tray 220. In this preferred embodiment there are two clamps, a first clamp 174A, which is fixedly mounted with respect to the tray 220, and a second clamp 174B, which is slidably mounted with respect to the tray 220. As described in more detail below, each of the clamps has two depending pins 226 and 228, and a depending drive spline 230. The tray 220 has a pair of holes 232 and 234 for receiving the pins 226 and 228 of clamp 174A, and a hole 236 for receiving the drive spline 230. Two mounting holes 238 and 240 allow the clamp 174A to be secured to the tray with screws 242 and washers 244. The tray 220 also has a pair of slots 246 and 248 for receiving the pins 226 and 228 of clamp 174B, and a slot 250 for receiving the drive spline 230. Two mounding slots 252 and 254 allow the clamp 174B to be secured to the tray with screws 242 and washers 244. The slots 246, 248, 250 and 252 and 254 allow the clamp to translate on the tray 220. A contact plate 192 is secured on the bottom of the tray for making electrical contact with the contact pad 194 on the device driver 52.

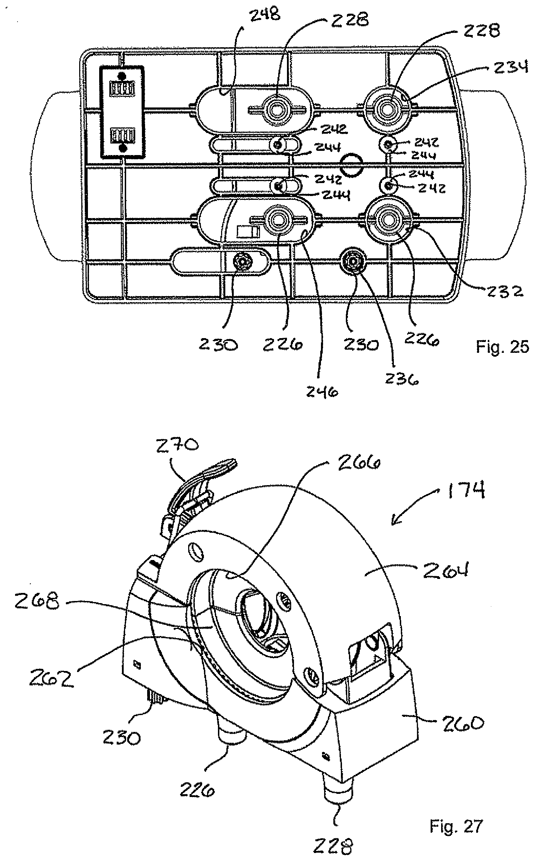

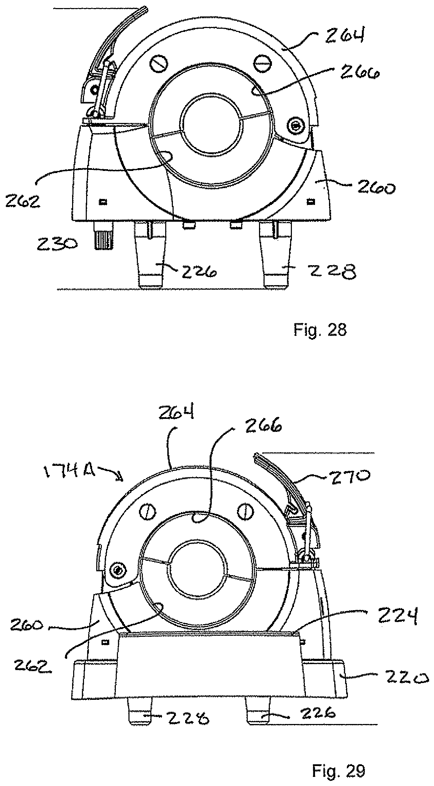

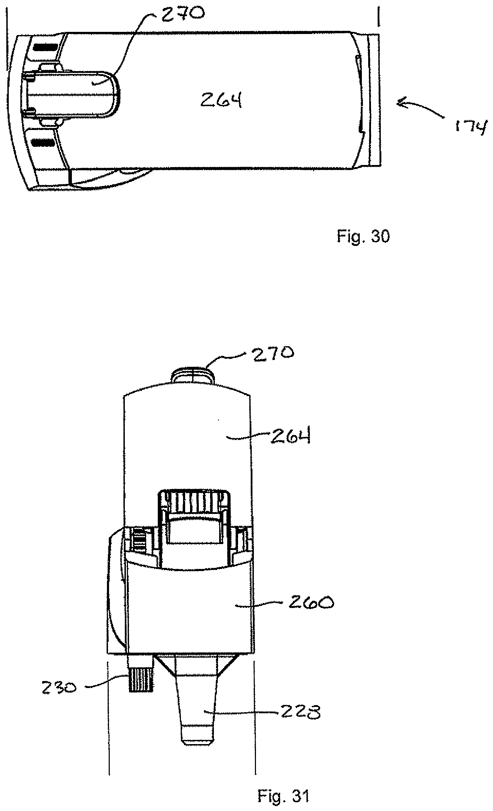

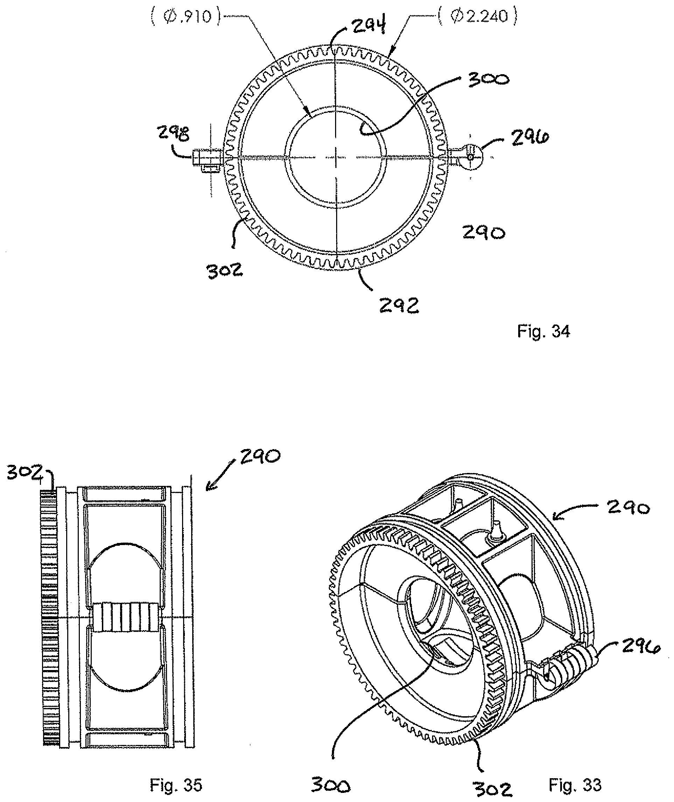

As shown in Figs. 27 - 36, each of the clamps 174 comprises a base 260 which carries the pins 226 and 228 and the drive spline 230. The base 260 has a semi-circular notch 262. A generally arcuate cover 264 has a semi-circular notch 266 therein which is hingedly attached at one end to the base 260, to pivot between an open position and closed position, in which the semi-circular notch 262 in the base 260 and the semi-circular notch 266 in the cover 264 form a generally circular opening 268 through the clamp. An over-center latch 270 releasably secures the other end of the cover 264 to the base 260 to retain the cover in its closed configuration.

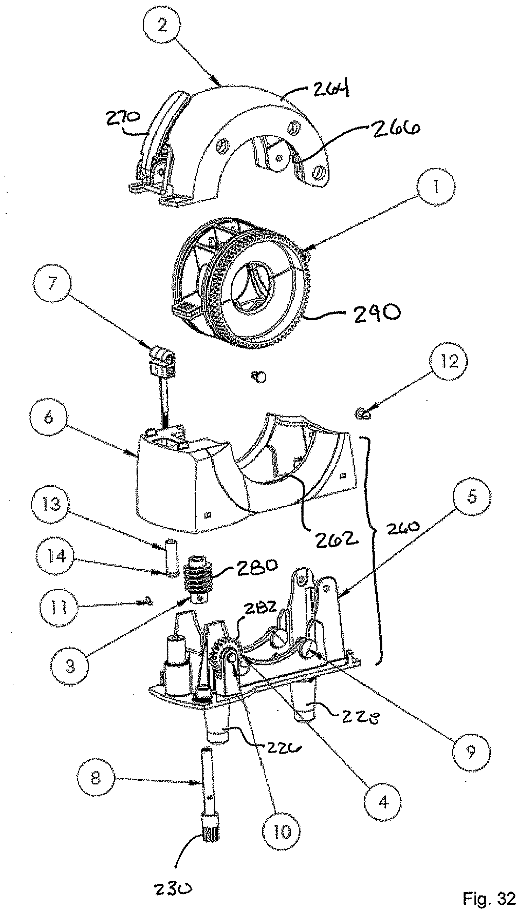

As best shown in Fig. 32, a worm gear 280 is mounted on the end of the spline 230 to rotate with the spline. The worm gear engages a sprocket 282, so that the sprocket turns when the spline 230 turns.

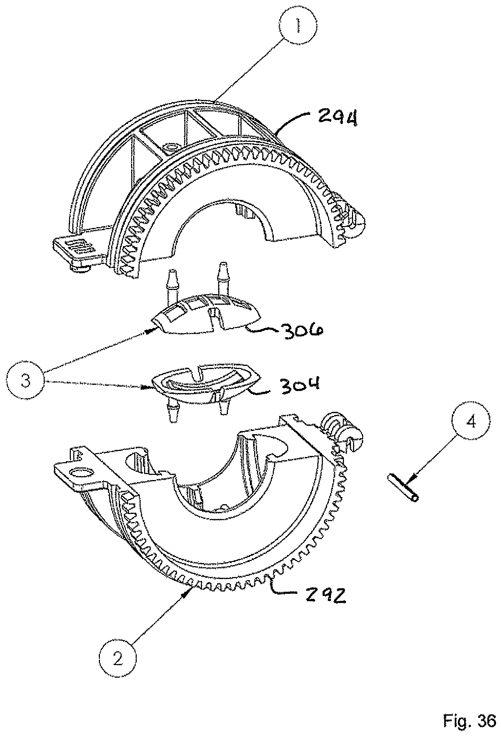

A split adapter ring 290 comprises first and second generally semi-circular halves 292 and 294, and has a hinged connection 296 at one side to pivot between an open position and a closed position in which the halves 292 and 294 form a ring. The other ends of the halves 292 and 294 are releasably connected, for example with a snap latch 298 to form a ring with a central opening 300. The split adapter ring 290 can be secured around a portion of the handle of a conventional manually operated medical device. The split adapter ring 290 has an associated ring gear 302 and is adapted to fit inside the clamp 174B, with the ring gear 302 engaged with the sprocket 282. The interior of each of the halves 292 and 294 can be specially adapted to receive the handle of a particular medical device, or various inserts (for example inserts 304 and 306 shown in Fig. 36) can be used to adapt a standardized halves to accommodate different medical devices.

### OPERATION

The operation of the preferred embodiment will be described with respect to a loop type EP Catheter, although, the invention is not so limited and embodiments of the remote manipulator can be used to operate a wide variety of medical devices which can be controlled through the manipulation of handle. The medical device has a handle with an actuator ring. Rotation of the actuator ring relative to the remainder of the handle causes the distal end of the loop catheter to bend. Translation of the actuator ring relative to the remainder of the handle causes the ring at the distal end to increase or decrease in size.

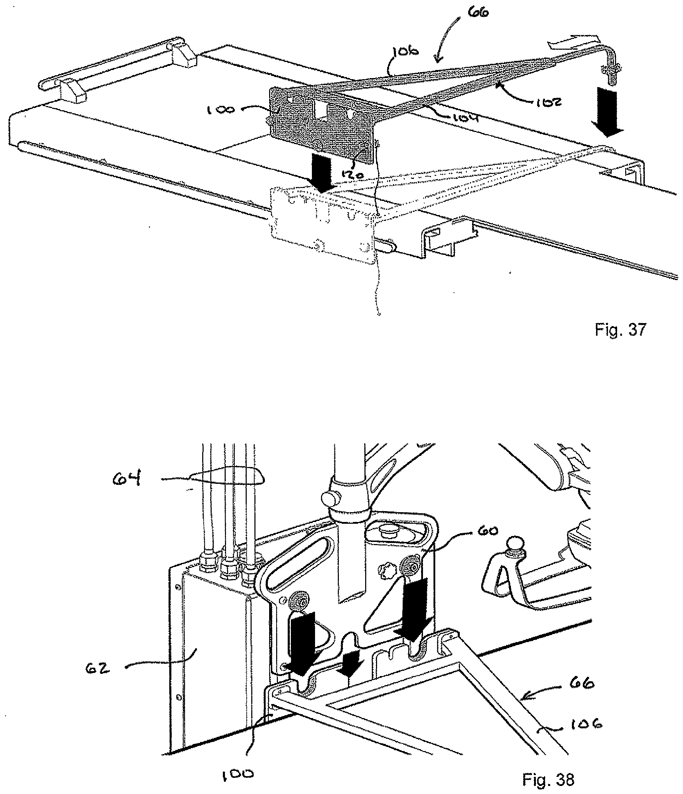

The system is first installed on a patient bed. As shown in Fig. 37, the platform 66 is laid across the surface of the bed, and secured to rails found on the sides of a typical patient bed. The cleats on the 112 and 114 on the base plate 100 are secured to the rail on one side of the bed by tightening the bolts 120 and 122. Similarly, on the opposite side of the bed, the cleat 124 on L-shaped bracket 110 is secured to the rail by tightening the bolt 128. Once the platform 66 has been secured on the patient bed, the bracket 60 is mounted on the platform 66. As shown in Fig. 38, the pins 94 and 96 on the bracket 60 fit into the semi-circular notches 130 and 132 in the support plate 100, and the pin 134 on the plate 100 engages the semi-circular notch 98 in the bracket 60. The bracket 60 and the support plate are secured by tightening bolt 136 on the bracket to engage the slot 138 in the base plate.

The device driver 52 is then positioned in the appropriate location by pivoting the articulating arm 56 around post 58, and securing it with bolt 76. The sections 70 and 72 can be moved and driver device 52 can be pivoted about the first, second, and third axes to bring the driver device to an appropriate position for conducting the procedure.

A surgical drape (not shown) in the form of an elongate plastic bag can be installed over the device driver 52 and articulating arm 56. The drape preferably has a puncturable window that generally corresponds in size and shape to the platform 204, and is aligned therewith. A replaceable disposable device interface 54, which can be provided in a sterile package, is removed from its sterile packaging and installed on the platform 204 of the device driver 52, with the pins 226 and 228 and spline 230 of each of the clamps 174 piercing the drape to connect to the sockets in the driver device. Alternatively, the window in the drape can be a framed opening, the tray 220 can seal with the frame, and the pins 226 and 228, and the spline 230 can engage their respective sockets without interference from the drape.

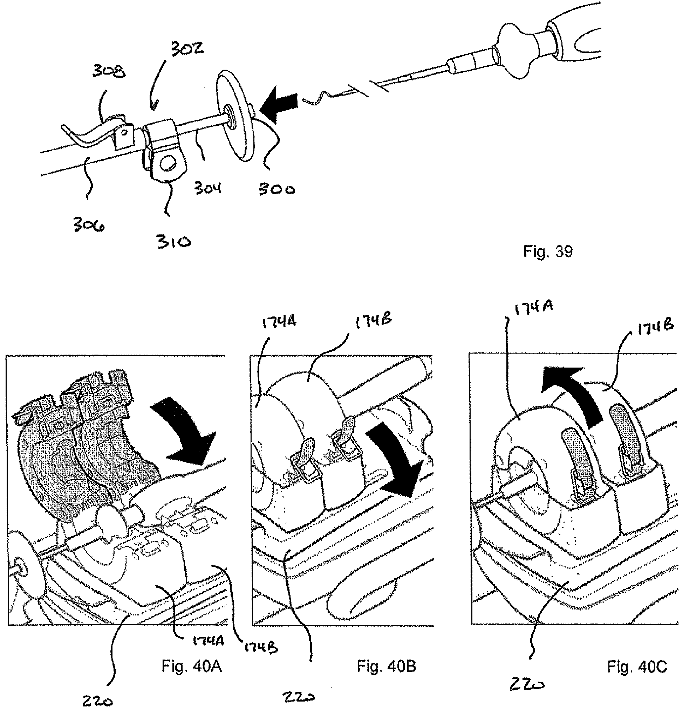

The elongate medical device is then prepared for use and mounting in the remote manipulator system. As shown in Fig. 39, the distal end of the medical device is introduced into the opening 300 in the proximal end of a telescoping catheter support 302. The telescoping catheter support 302 comprises at least two relatively telescoping tubes 304 and 306 with a lock 308 for locking the tubes 304 and 306 in position to set the length of the support. The distal end of the support 302 engages the introducer sheath. A clip 310 allows the proximal end of the catheter support 302 to engage the mount 168.

As shown in Figs. 40A through 40C, the clamps 174A and 174B are opened, and the handle of the medical device placed in the clamps, with the actuator ring of the medical device aligned with clamp 174A, and the remainder of the handle aligned with clamp 174B. The covers 264 of the clamps 174A and 174B are closed, and the latches 270 engaged.

Once the medical device is engaged in the remote manipulator system, the distal end of the medical device can be introduced into the body and manipulated with the remote manipulation system 50.

When it is desired to advance the catheter, the drive 148 is actuated which causes the translation mechanism 212 to advance the body 170 of the device driver 52 relative to the base 160, which advances the catheter mounted thereon. When it is desired to retract the catheter, the drive 148 is actuated which causes the translation mechanism 212 to retract the body 170 of the device driver relative to its base 160. When it is desired to rotate the distal end of the catheter, the drives 142 and 146 are operated to operate transmissions 206 and 310 which cause the splines 230 of both of the clamps 174A and 174B to turn, thereby turning the split adapter ring gears 302 and thus, the entire device engaged therein. When it is desired to operate the actuation ring on the handle of the device, relative translation of the ring and the remainder of the handle can be caused by operating the drive 144 to operate the translation mechanism 208 to cause the clamp 174B engaging the handle to move relative to the clamp 174A engaging the actuator ring, to thereby cause relative movement between the actuator ring and the handle. Relative rotation of the actuator ring and the remainder of the handle can be caused by operating the drive 142 to operate transmission 206 to operate the spline 230 of clamp 174A, rotating the actuator ring relative to the remainder of the handle, or operating drive 210 to operate transmission 210 to operate the spline 230 of clamp 174B rotating the remainder of the handle relative to the actuator ring, or to operate drives 142 and 146 and different rates and or in different directions to cause relative rotation between the actuator ring engaged in clamp 174A and the remainder of the handle engaged in 174B.

The drives 142, 144, 146, and 148 can be under direct control by a physician through a suitable interface, or the drives can be under the control of a microprocessor under the supervision and direction of a physician.

In an emergency, the clamps 174A and 174B can be easily opened by operating latches 270 to release the cover and pulling the medical device free. The split adapter rings can be easily removed from the device so that the device can be used manually.

Embodiments of the remote manipulator device can be adapted to a wide variety of medical devices to allow the devices to be positioned and operated inside the body under remote control by a physician, or by physician-supervised computer control.

## Claims

1. A system for remotely manipulating an elongate medical device of the type having a distal end adapted to be introduced into a patient and a handle at the proximal end with rotatable and translatable controls for manipulating the device, the system comprising:
a device driver (52) positionable adjacent the patient;
a device interface (54) releasably mounted on the device driver (52); and
a control for selectively operating the device driver (52) to advance and retract the device driver (52) relative to the patient to advance and retract the distal end of the medical device in the patient; wherein the device interface (54) comprises a first clamp (174a) for releasably engaging a first portion of the handle of the medical device and a second clamp (174b) for releasably engaging a second portion of the handle of the medical device; and in that the control is configured for selectively operating the device driver (52) to cause the first and second clamps (174a, 174b) to rotate the first and second portions of the handle of the medical device to rotate the distal end of the medical device in the patient; for selectively operating the device driver (52) to move the second clamp (174b) relative to the first clamp (174a) to cause relative translation between the first portion of the handle and the second portion of the handle to thereby operate the translatable control on the handle; and for selectively operating the device driver (52) to cause the at least one of the first and second clamps (174a, 174b) to rotate the portion of the medical device releasably engaged therein, to cause relative rotation between the first portion of the handle and the second portion of the handle to thereby operate the rotatable control on the handle.

2. A system according to claim 1 wherein each of the first and second clamps (174a, 174b) comprises a base (260) having a concave recess (262), a hinged cover (264) having a concave recess (266), the cover (264) being pivotally operable between an open positon in which a portion of the handle of a medical device can be inserted and removed from the clamp (174a), and a closed position in which the concave recesses (262, 266) in the base (260) and cover (264) cooperate to create a central opening (268) for receiving and retaining the portion of the handle of the medical device inserted therein; and a latch (270) for releasably securing the cover (264) in its closed position.

3. A system according to claim 2 wherein each of the clamps (174a, 174b) further comprises an adapter ring (290), rotably mountable in the central opening (268) between the cover (262) and the base (260), and comprise first and second hingedly connected portions (292, 294) adapted to enclose a portion of the handle of the medical device.

4. A system according to claim 3 wherein the adapter ring (290) includes a ring gear (302), and wherein the base (260) includes a gear train (282) engaging the ring gear (302) on the adapter ring (290) and engagable with the device driver (52) to turn the adapter ring (290) within the clamp (174a, 174b).

5. A system according to claim 1 wherin the device interface (54) comprises a tray (220), a first clamp (174a) mounted on the tray (220) in a fixed position, and a second clamp (174b) slidably mounted on the tray (220), the first and second clamps (174a, 174b) preferably having portions (226, 228) extending through the tray (220), adapted to engage portions of the device driver (52).

6. A system according to claim 1, wherein the device interface (54) comprises a tray (220); wherein the first and second clamps (174a, 174b) include a rotatble adapter ring (290) for engaging the respective portions of the device handle; wherein the first clamp (174a) is fixedly engaged on the tray (220) and the second clamp (174b) is slidably mounted on the tray (220); and wherein each of the clamps (174a, 174b) includes a portion (226, 228) extending through the tray (220) and engaging the device driver (52), and a gear train (282) engagable with the device driver (52) for rotating the adapter (290) of the clamps (174a, 174b).

7. A system according to claim 6 wherein the device driver (52) comprises a base (160); a body (170), a translation mechanism for translating the body (170) relative to the base (160); a transmission for engaging a portion of the first clamp (174a) mounted thereon to operate the gear train (282) to rotate the adapter ring (290) of the first clamp (174a); a translation mechanism engaging a portion of the second clamp for translating the second clamp (174b) relative to the first clamp (174a); and a transmission for engaging a portion of the second clamp (174b) mounted thereon to operate the gear train (282) to rotate the adapter ring (290) of the second clamp (174b).

8. A system according to claim 6, wherein the first clamp (174a) comprises a base (260) and a hinged cover (264); and wherein the adapter ring (290) is a split adapter ring, rotatably mounted in the base (260) and cover (264), adapted to receive the first portion of the handle of the medical device, and a gear train (282) for rotating the split adapter ring (290), the gear train (282) having a projecting spline adapted to engage a socket on a device driver (52) on which the device interface (54) is mounted so that the device driver (52) can rotate the split ring adapter (290) of the first clamp (174a); and wherein the second clamp (174b) comprises a base (260) and a hinged cover (264); and wherein the adapter ring (290) is a split adapter ring, rotatably mounted in the base (260) and cover (264), adapted to receive the second portion of the handle of the medical device, and the gear train (282) for rotating the split adapter ring (290), the gear train (282) having a projecting spline adapted to engage a socket on a device driver (52) on which the device interface (54) is mounted so that the device driver (52) can rotate the split ring adapter (290) of the second clamp (174b), the second clamp (174b) having a depending portion (226, 228) adapted to engage a translation mechanism on a device driver (52) on which the device interface (54) is mounted so that the device driver (52) can translate the second clamp (174b) relative to the first clamp (174a).

## Patentansprüche

1. Ein System für das fernbediente Betätigen einer länglichen medizinischen Vorrichtung des Typs mit einem distalen Ende, das dafür ausgelegt ist, um in einen Patienten eingeführt zu werden, und einem Griff an dem proximalen Ende mit drehbaren und übersetzbaren Bedienelementen zum Betätigen der Vorrichtung, das System umfassend:
ein Vorrichtungsantrieb (52), der neben dem Patienten positioniert werden kann;
eine Vorrichtungsschnittstelle (54), die abnehmbar an dem Vorrichtungsantrieb (52) montiert ist; und
ein Bedienelement, um wahlweise den Vorrichtungsantrieb (52) zu betätigen und den Vorrichtungsantrieb (52) im Verhältnis zum Patienten vor oder zurückzufahren, um das distale Ende der medizinischen Vorrichtung im Patienten vor und zurückzufahren; wobei die Vorrichtungsschnittstelle (54) einen ersten Bügel (174a) umfasst, um einen ersten Teil des Griffs der medizinischen Vorrichtung abnehmbar einrasten zu lassen, und einen zweiten Bügel (174b), um einen zweiten Teil des Griffs der medizinischen Vorrichtung abnehmbar einrasten zu lassen; und wobei das Bedienelement konfiguriert ist, wahlweise den Vorrichtungsantrieb (52) zu betätigen und den ersten Bügel und den zweiten Bügel (174a, 174b) zu veranlassen, den ersten und den zweiten Teil des Griffs der medizinischen Vorrichtung zu drehen und das distale Ende der medizinischen Vorrichtung in dem Patienten zu drehen; um wahlweise den Vorrichtungsantrieb (52) zu betätigen und den zweiten Bügel (174b) im Verhältnis zum ersten Bügel (174a) zu bewegen, um die relative Übersetzung zwischen dem ersten Teil des Griffs und dem zweiten Teil des Griffs zu veranlassen, dadurch das übersetzbare Bedienelement des Griffs zu betätigen; und um wahlweise den Vorrichtungsantrieb (52) zu betätigen, um mindestens entweder den ersten oder den zweiten Bügel (174a, 174b) zu veranlassen, den Teil der medizinischen Vorrichtung zu drehen, der abnehmbar daran befestigt ist, und eine relative Drehung zwischen dem ersten Teil des Griffs und dem zweiten Teil des Griffs zu veranlassen, dadurch das drehbare Bedienelement des Griffs zu betätigen.

2. Ein System nach Anspruch 1, wobei der erste und der zweite Bügel (174a, 174b) einen Sockel (260) mit einer konkaven Aussparung (262) und eine klappbare Abdeckung (264) mit einer konkaven Aussparung (266) umfassen, die Abdeckung (264) ist drehbar zwischen einer offenen Position einstellbar, in der ein Teil des Griffs einer medizinischen Vorrichtung in den Bügel (174a) eingesetzt oder daraus entnommen werden kann, und einer geschlossenen Position, in der die konkaven Aussparungen (262, 266) in dem Sockel (260) und an der Abdeckung (264) zusammenwirken, um eine zentrale Öffnung (268) zu bilden, um den Teil des Griffs der darin eingesetzten medizinischen Vorrichtung aufzunehmen und zu halten; und einen Riegel (270) für die abnehmbare Sicherung der Abdeckung (264) in ihrer geschlossenen Position.

3. Ein System nach Anspruch 2, wobei jeder der Bügel (174a, 174b) darüber hinaus einen Adapterring (290) umfasst, der drehbar in der zentralen Öffnung (268) zwischen der Abdeckung (262) und dem Sockel (260) montiert werden kann, und einen ersten und zweiten klappbar verbundenen Teil (292, 294) umfasst, der dafür ausgelegt ist, einen Teil des Griffs der medizinischen Vorrichtung zu umschließen.

4. Ein System nach Anspruch 3, wobei der Adapterring (290) einen Zahnkranz (302) einschließt, und wobei der Sockel (260) eine Zahnradübersetzung (282) einschließt, die den Zahnkranz (302) auf dem Adapterring (290) hält und mit dem Vorrichtungsantrieb (52) verbunden werden kann, um den Adapterring (290) in dem Bügel (174a, 174b) zu drehen.

5. Ein System nach Anspruch 1, wobei die Vorrichtungsschnittstelle (54) eine Ablage (220), einen ersten Bügel (174a), der auf der Ablage (220) in einer festen Position montiert ist, und einen zweiten Bügel (174b) umfasst, der verschiebbar auf der Ablage (220) montiert ist, wobei der erste und der zweite Bügel (174a, 174b) vorzugsweise Teile (226, 228) aufweisen, die sich durch die Ablage (220) erstrecken, und dafür ausgelegt sind, Teile des Vorrichtungsantrieb (52) zu halten.

6. Ein System nach Anspruch 1, wobei die Vorrichtungsschnittstelle (54) eine Ablage (220) umfasst; wobei der erste und der zweite Bügel (174a, 174b) einen drehbaren Adapterring (290) einschließen, um die jeweiligen Teile des Vorrichtungsgriffs zu befestigen; wobei der erste Bügel (174a) fest mit der Ablage (220) verbunden ist und der zweite Bügel (174b) verschiebbar auf der Ablage (220) montiert ist; und wobei jeder der Bügel (174a, 174b) einen Teil (226, 228) einschließt, der sich durch die Ablage (220) erstreckt und den Vorrichtungsantrieb (52) hält, und eine Zahnradübersetzung (282), die mit dem Vorrichtungsantrieb (52) verbunden werden kann, um den Adapter (290) der Bügel (174a, 174b) zu drehen.

7. Ein System nach Anspruch 6, wobei der Vorrichtungsantrieb (52) einen Sockel (160) umfasst; ein Gehäuse (170), einen Übersetzungsmechanismus zum Übersetzen des Gehäuses (170) im Verhältnis zum Sockel (160); ein Getriebe, um in einen Teil des ersten Bügels (174a) einzurasten, der darauf montiert ist, um die Zahnradübersetzung (282) zu betätigen und den Adapterring (290) des ersten Bügels (174a) zu drehen; einen Übersetzungsmechanismus, der in einen Teil des zweiten Bügels einrastet, um den zweiten Bügel (174b) im Verhältnis zum ersten Bügel (174a) zu übersetzen; und ein Getriebe, um in einen Teil des zweiten Bügels (174b) einzurasten, der darauf montiert ist, um die Zahnradübersetzung (282) zu betätigen und den Adapterring (290) des zweiten Bügels (174b) zu drehen.

8. Ein System nach Anspruch 6, wobei der erste Bügel (174a) einen Sockel (260) und eine klappbare Abdeckung (264) umfasst; und wobei der Adapterring (290) ein geteilter Adapterring ist, der drehbar an dem Sockel (260) und der Abdeckung (264) montiert ist, und dafür ausgelegt ist, den ersten Teil des Griffs der medizinischen Vorrichtung aufzunehmen, sowie eine Zahnradübersetzung (282) zum Drehen des geteilten Adapterrings (290), wobei die Zahnradübersetzung (282) einen hervorstehenden Keil aufweist, der dafür ausgelegt ist, eine Buchse an einem Vorrichtungsantrieb (52) einrasten zu lassen, auf dem die Vorrichtungsschnittstelle (54) montiert ist, sodass der Vorrichtungsantrieb (52) den geteilten Adapterring (290) des ersten Bügels (174a) drehen kann; und
wobei der zweite Bügel (174b) einen Sockel (260) und eine klappbare Abdeckung (264) umfasst; und wobei der Adapterring (290) ein geteilter Adapterring ist, der drehbar an dem Sockel (260) und der Abdeckung (264) montiert ist, und dafür ausgelegt ist, den zweiten Teil des Griffs der medizinischen Vorrichtung und die Zahnradübersetzung (282) zum Drehen des geteilten Adapterrings (290) aufzunehmen, wobei die Zahnradübersetzung (282) einen hervorstehenden Keil aufweist, der dafür ausgelegt ist, eine Buchse an einem Vorrichtungsantrieb (52) einrasten zu lassen, auf dem die Vorrichtungsschnittstelle (54) montiert ist, sodass der Vorrichtungsantrieb (52) den geteilten Adapterring (290) des zweiten Bügels (174b) drehen kann, wobei der zweite Bügel (174b) einen abhängigen Teil (226, 228) aufweist, der dafür ausgelegt ist, einen Übersetzungsmechanismus an einem Vorrichtungsantrieb (52) zu halten, auf dem die Vorrichtungsschnittstelle (54) montiert ist, sodass der Vorrichtungsantrieb (52) den zweiten Bügel (174b) im Verhältnis zum ersten Bügel (174a) übersetzen kann.

## Revendications

1. Système pour la manipulation à distance d'un dispositif médical allongé du type doté d'une extrémité distale adaptée pour être introduite dans un patient et une poignée au niveau de l'extrémité proximale avec des commandes de rotation et de translation pour la manipulation du dispositif, le système comprenant :
un pilote de dispositif (52) positionnable de manière adjacente au patient ;
une interface du dispositif (54) montée de manière amovible sur le pilote de dispositif (52) ; et
une commande pour un fonctionnement sélectif du pilote de dispositif (52) pour faire avancer et reculer le pilote de dispositif (52) par rapport au patient pour faire avancer et reculer l'extrémité distale du dispositif médical dans le patient ;
dans lequel l'interface du dispositif (54) comprend une première pince (174a) pour engager de façon libérable une première partie de la poignée du dispositif médical et une deuxième pince (174b) pour engager de façon libérable une deuxième partie de la poignée du dispositif médical ; et dans lequel la commande est configurée pour un fonctionnement sélectif du pilote de dispositif (52) pour amener les première et deuxième pinces (174a, 174b) à faire pivoter les première et deuxième parties de la poignée du dispositif médical pour pivoter l'extrémité distale du dispositif médical dans le patient ; pour un fonctionnement sélectif du pilote de dispositif (52) pour déplacer la deuxième pince (174b) par rapport à la première pince (174a) pour provoquer une translation relative entre la première partie de la poignée et la deuxième partie de la poignée pour ainsi faire fonctionner la commande de translation sur la poignée ; et pour un fonctionnement sélectif du pilote de dispositif (52) pour amener l'au moins une parmi les première et deuxième pinces (174a, 174b) à faire pivoter la partie du dispositif médical engagée de façon libérable dedans, pour provoquer une rotation relative entre la première partie de la poignée et la deuxième partie de la poignée pour ainsi faire fonctionner la commande de rotation sur la poignée.

2. Système selon la revendication 1, dans lequel chacune des première et deuxième pinces (174a, 174b) comprend une base (260) ayant un renfoncement concave (262), un couvercle à charnières (264) ayant un renfoncement concave (266), le couvercle (264) étant utilisable en pivotement entre une position ouverte dans laquelle une partie de la poignée d'un dispositif médical peut être insérée et déplacée de la pince (174a), et une position fermée dans laquelle les renfoncements concaves (262, 266) dans la base (260) et le couvercle (264) se mettent ensemble pour créer une ouverture centrale (268) pour la réception et la conservation de la partie de la poignée du dispositif médical insérée dedans ; et un loquet (270) pour fixer de manière libérable le couvercle (264) dans sa position fermée.

3. Système selon la revendication 2, dans lequel chacune des pinces (174a, 174b) comprend en outre une bague d'adaptateur (290), montable de manière pivotable dans l'ouverture centrale (268) entre le couvercle (262) et la base (260), et comprend des première et deuxième parties reliées par charnière (292, 294) adaptées pour fermer une partie de la poignée du dispositif médical.

4. Système selon la revendication 3, dans lequel la bague d'adaptateur (290) inclut un engrenage annulaire (302), et dans lequel la base (260) inclut un train d'engrenages (282) engageant l'engrenage annulaire (302) sur la bague d'adaptateur (290) et pouvant venir en prise avec le pilote de dispositif (52) pour tourner la bague d'adaptateur (290) dans la pince (174a, 174b).

5. Système selon la revendication 1, dans lequel l'interface du dispositif (54) comprend une plaque (220), une première pince (174a) montée sur la plaque (220) dans une position fixe, et une deuxième pince (174b) montée de manière coulissante sur la plaque (220), les première et deuxième pinces (174a, 174b) ayant de préférence des parties (226, 228) s'étendant à travers la plaque (220), adapté pour engager des parties du pilote de dispositif (52).

6. Système selon la revendication 1, dans lequel l'interface du dispositif (54) comprend une plaque (220) ; dans lequel les première et deuxième pinces (174a, 174b) incluent une bague d'adaptateur rotative (290) pour engager les parties respectives du dispositif poignée ; dans lequel la première pince (174a) vient en prise de manière fixe sur la plaque (220) et la deuxième pince (174b) est montée de manière coulissante sur la plaque (220) ; et dans lequel chacune des pinces (174a, 174b) inclut une partie (226, 228) s'étendant à travers la plaque (220) et engageant le pilote de dispositif (52), et un train d'engrenages (282) pouvant venir en prise avec le pilote de dispositif (52) pour la rotation de l'adaptateur (290) des pinces (174a, 174b).

7. Système selon la revendication 6, dans lequel le pilote de dispositif (52) comprend une base (160) ; un corps (170), un mécanisme de translation pour la translation du corps (170) par rapport à la base (160) ; une transmission pour engager une partie de la première pince (174a) montée dessus pour faire fonctionner le train d'engrenages (282) pour pivoter la bague d'adaptateur (290) de la première pince (174a) ; un mécanisme de translation engageant une partie de la deuxième pince pour la translation de la deuxième pince (174b) par rapport à la première pince (174a) ; et une transmission pour engager une partie de la deuxième pince (174b) montée dessus pour faire fonctionner le train d'engrenages (282) pour pivoter la bague d'adaptateur (290) de la deuxième pince (174b).

8. Système selon la revendication 6, dans lequel la première pince (174a) comprend une base (260) et un couvercle à charnières (264) ; et dans lequel la bague d'adaptateur (290) est une bague d'adaptateur fendue, montée de manière rotative dans la base (260) et le couvercle (264), adaptée pour recevoir la première partie de la poignée du dispositif médical, et un train d'engrenages (282) pour la rotation de la bague d'adaptateur fendue (290), le train d'engrenages (282) ayant une cannelure faisant saillie adaptée pour engager une prise sur un pilote de dispositif (52) sur lequel l'interface du dispositif (54) est monté de sorte que le pilote de dispositif (52) peut faire pivoter la bague d'adaptateur fendue (290) de la première pince (174a) ; et
dans lequel la deuxième pince (174b) comprend une base (260) et un couvercle à charnières (264) ; et dans lequel la bague d'adaptateur (290) est une bague d'adaptateur fendue, montée de manière rotative dans la base (260) et le couvercle (264), adaptée pour recevoir la deuxième partie de la poignée du dispositif médical, et le train d'engrenages (282) pour la rotation de la bague d'adaptateur fendue (290), le train d'engrenages (282) ayant une cannelure faisant saillie adaptée pour engager une prise sur un pilote de dispositif (52) sur lequel l'interface du dispositif (54) est montée de sorte que le pilote de dispositif (52) peut faire pivoter la bague d'adaptateur fendue (290) de la deuxième pince (174b), la deuxième pince (174b) ayant une partie dépendante (226, 228) adaptée pour engager un mécanisme de translation sur un pilote de dispositif (52) sur lequel l'interface du dispositif (54) est monté de sorte que le pilote de dispositif (52) peut réaliser une translation de la deuxième pince (174b) par rapport à la première pince (174a).
